(19) **Europäisches Patentamt**
**European Patent Office**
**Office européen des brevets**

(11) **EP 2 896 371 A1**

(12) **EUROPEAN PATENT APPLICATION**

(43) Date of publication:
**22.07.2015 Bulletin 2015/30**

(51) Int Cl.:
*A61B 8/08* (2006.01)     *G06T 5/00* (2006.01)
*G06T 7/00* (2006.01)     *A61B 8/00* (2006.01)

(21) Application number: **14151886.0**

(22) Date of filing: **21.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Her Majesty The Queen, In Right Of Canada, As**
**Represented by the Minister of National Defence**
**Ottawa, ON K1A 0K2 (CA)**

(72) Inventors:
• **Stergiopoulos, Stergios**
**TORONTO, Ontario ON M2P 1B6 (CA)**

• **Shek, Pang**
**TORONTO, Ontario ON M2N 1P4 (CA)**
• **Plataniotis, Konstantinos**
**TORONTO, Ontario ON M6B 1Z4 (CA)**
• **Marsousi, Mahdi**
**TORONTO, Ontario ON M2M 4M1 (CA)**

(74) Representative: **Maillet, Alain**
**Cabinet Le Guen Maillet**
**5, place Newquay**
**B.P. 70250**
**35802 Dinard Cedex (FR)**

(54) **Computer aided diagnosis for detecting abdominal bleeding with 3D ultrasound imaging**

(57) The presented invention provides a detection method for free fluid in the human body. The preprocessing procedure is applied to reduce the speckle noise and enhance tissue volumetric pixels (voxels) intensity levels to make tissue and non-tissue voxels more distinguishable from each other. An initial surface selection step provides flexibility to either manually or automatically selecting a seed point to segment fluid regions in the volumetric data. A kidney and liver organ detection procedure provides a method for determining whether the detected fluid region is a normal fluid-carrying body organ or if it is due to a medical condition as a free fluid region. A combinational approach using a 3-dimensional Snake and Level-Set is utilized to accurately detect fluid regions in the volumetric ultrasound data.

Figure 1

**Description**

The present invention relates to a diagnosis system for analyzing 3D ultrasound images to detect the size and location of internal organs and free fluid.

The rapid diagnosis of invisible internal injury in austere and hostile front-line environments remains a challenge for medical personnel. The availability of a portable real-time 3-Dimensional (3D) ultrasound imaging system with automated diagnostic capabilities for detecting non-visible internal abdominal bleeding, pneumothorax, hematothorax and facilitating image guided operations is helpful, if not essential, in supporting triage and medical decisions for trauma patients.

In trauma patients, inner-body fluids can accumulate at the lowest position of the concavities within the body. The site of fluid accumulation depends on the source of the bleeding and the position of the patient. In general, patients are examined on the bed lying flat on the back. In such conditions, inner-body fluids accumulate at positions between the liver and the kidneys, along the spleen border, at the position posterior to the bladder or uterus, and in the pericardial space. Similarly, there are conditions akin to internal bleeding which also cause an accumulation of fluid in a patient's internal cavities. For these non-trauma conditions, being able to detect such fluid accumulation would also be greatly helpful.

One system developed to address the concerns of internal bleeding and the fluid accumulation it causes is ultrasound imaging. Ultrasound imaging maps out ultrasound reflection points in order to build up an internal image of the target. These systems return information about the internal structure of a target. In general, ultrasound refers to high frequency longitudinal mechanical waves, more commonly known as megahertz (MHz) sound waves. Ultrasound can propagate in any physical medium but has better propagation characteristics when traveling through solid or liquid media. Intermolecular coupling in solids and liquids affects the rate at which mechanical waves propagate. A solid with strong intermolecular coupling allows for faster ultrasound propagation compared to a low density fluid with weak coupling.

Currently, inner-body fluids are detected by scanning the human body on specific characteristic locations by means of the so-called "Focus Assessment with Sonography in Trauma" (FAST) method. FAST is an important method used to identify free intraperitoneal, intrathoracic, or pericardial fluid. FAST is primarily used at the patient's bedside by emergency physicians and trauma surgeons. The development of hand-held ultrasound devices facilitated the introduction of FAST into pre-hospital trauma management (p-FAST).

FAST consists of multiple, focused, ultrasonographic views of the abdomen and the pericardium. The use of multiple views increases the sensitivity of the FAST examination in the detection of hemoperitoneum.

Currently, there exist other known imaging systems that use 3-Dimensional ultrasound systems. For instance, United States (U.S.) patent US7920731 provides a method for differentiating between a blood vessel bifurcation and a bleeding blood vessel in an ultrasound volume. This method uses Doppler waveform data to determine if the bifurcation is a point of internal bleeding.

Additionally, U.S. patent US7803116 discloses an ultrasound-based method for detecting and imaging vibrations in tissue. The tissue vibrations are analyzed as a detection method for internal bleeding.

Other examples of internal or ultrasound imaging systems and methods include U.S. patent US8520947, U.S. patent US6561980, U.S. patent US6385332, U.S. patent US6719696, U.S. patent US6482160, and U.S. patent application 13/743490.

However, none of the above disclosures provides a portable real-time 3D ultrasound imaging system with automated diagnostic capabilities for detecting non-visible internal abdominal bleeding. There is therefore a need for a non-invasive medical detection system to address casualty care support.

The present invention provides a computer aided diagnosis system that uses 3D ultrasound imaging to detect human internal organs such as kidneys, livers, spleens and free fluid (i.e. fluids due to internal bleeding). The system detects and labels internal organs to assist the user in locating the ultrasound probe position. Second, inner body fluids are detected by the system when these appear as dark areas in 3D ultrasound images.

In a first aspect, the present invention provides a method for detecting an area of fluid in volumetric ultrasound data derived from an image of a mammal's body part, the method comprising:

     a) receiving volumetric data;
     b) denoising said data to result in denoised data;
     c) enhancing a contrast of tissue voxels in said denoised data;
     d) determining an initial surface of a volume presented as a dark area in said denoised data;
     e) determining if an internal organ is detected in said volume in said denoised data; and
     f) isolating said volume in said denoised data to determine a size and location of said volume.

In another aspect, the present invention provides computer readable media having encoded thereon computer readable and computer executable instructions which, when executed, implements a method for detecting an area of fluid in volumetric data derived from an image of a mammal's body part, the method comprising:

a) receiving volumetric data;
b) denoising said data to result in denoised data;
c) enhancing a contrast of tissue voxels in said denoised data;
d) determining an initial surface of a volume presented as a dark area in said denoised data;
e) determining if an internal organ is detected in said volume in said denoised data; and
f) isolating said volume in said denoised data to determine a size and location of said volume.

The embodiments of the present invention will now be described by reference to the following figures, in which identical reference numerals in different figures indicate identical elements and in which:

FIGURE 1 is a block diagram detailing the steps in a de-speckling method used in one aspect of the present invention;
FIGURE 2 is a block diagram detailing the steps in a tissue volumetric pixels (voxels) intensity enhancement method;
FIGURE 3 is a block diagram of the steps in an initial surface selection procedure using both manual and automatic methods;
FIGURE 4 is a block diagram illustrating the steps for a process for detecting kidney and liver organs in volumetric data;
FIGURE 5 is a table showing an internal force relation between each surface sample point with its neighbours as parametric coefficients derived by the discretized Euler-Lagrange equation;
FIGURE 6 is a block diagram of the steps in a fluid segmentation procedure using a combination of 3D Snake and Level-Set methods;
FIGURE 7 is a block diagram of the steps in a procedure for determining a medical condition of a patient based on volumetric ultrasound data according to one aspect of the invention; and
FIGURE 8 illustrates a view panel of a developed Graphical User Interface (GUI).

The Figures are not to scale and some features may be exaggerated or minimized to show details of particular elements while related elements may have been eliminated to prevent obscuring novel aspects. Therefore, specific structural and functional details disclosed herein are not to be interpreted as limiting but merely as a basis for the claims and as a representative basis for teaching one skilled in the art to variously employ the present invention.

The task of object detection in 3D medical images has been investigated by researchers, due to its implications for medical diagnosis. The procedural steps of object detection in 3D volumes consist of preprocessing tasks, manual adjustment, 3D segmentation and classification. The aim of the preprocessing tasks is to put more emphasis on valuable information and to reduce the effect of unwanted interfering signals. Some examples of such tasks are denoising, edge refinement, contrast enhancement and volume clipping. As a second step, user intervention can be applied to boost the segmentation results. The 3D segmentation task can be considered to be the main part of an anatomical organ detection procedure. The selected 3D segmentation method for ultrasound volumes has to be robust against intensity variability, speckle noise and discontinuities among object walls. Finally, the classification step labels the segmented region as internal bleeding area, kidney, liver or other organs of interest.

There are numerous approaches to denoising images in order to reduce the speckle noise in ultrasound images. Some of these approaches include Temporal Averaging, Homomorphic Wiener Filtering, Median Filtering, Bayesian Denoising and Wavelet Thresholding. All of these methods result in the loss of information and the blurring of edges.

The Speckle Reducing Anisotropic Diffusion (SRAD) was proposed by Yongjian and Action. In this method, intensities are diffused based on the gradient magnitude edge and speckle noise level. At edge regions, intensities become 0, whereas at homogeneous regions, intensities become 1 and diffusion is performed. In another method called Squeeze Box Filter (SBF), outliers are suppressed as a local mean of their neighbourhood.

Yet another approach to de-speckle ultrasound images is to apply the sparse representation framework to represent the signal while suppressing noise. The double-sparsity method considers a sparse representation for the learned dictionary based on an analytical dictionary, such as a Discrete Cosine Transform (DCT) dictionary. This method uses a dictionary for the denoising ultrasound volume and sparsely represents the volume to effectively reduce the noise level.

Additionally, much research has been dedicated to segment 3D ultrasound images, such as Level-Set based approaches, region growing segmentation, Watershed and Graph-cuts methods, and Snake active contour models. The task of ultrasound image segmentation is known to be highly challenging, due to innate problems of ultrasound images including high speckle noise, inconsistent intensity levels, and discontinuities in regions and boundaries.

The 3D Snake approach provides a highly robust method against boundaries' discontinuities, if its parameters are correctly adjusted. However, the precise segmentation using the 3D Snake method requires an extensive number of sample points, resulting in a very high computational cost.

Region growing segmentation methods employ iterative propagation of an initiated region into homogenous regions that have similar intensity levels. This method is highly sensitive to the intensity variations and fails to correctly operate in the ultrasound volumes which are naturally varying in intensity.

The simplicity of extending the Level-Set approach from 2-Dimensional (2D) to 3D-segmentation contributes to this approach being commonly used in 3D medical image processing. It provides an accurate solution for some medical imaging modalities such as Magnetic Resonance Imaging (MRI) and Computed Tomography (CT). However, in the case of ultrasound images, the Level-Set method can fail in the presence of discontinuities among boundaries.

In one aspect, the present invention is a computer aided diagnosis system that uses, as input, 3D ultrasound images to detect human internal organs, such as kidneys, livers and spleens, as well as free fluid, i.e. fluid due to internal bleeding. The present invention facilitates automatic object recognition for non-skilled users. A person skilled in the art will understand that the method steps of the present invention may be programmed into a handheld device, or any other suitable data processing device.

The present invention aims to detect and label internal organs in order to locate the ultrasound probe position. Another aim is to detect inner body fluids presented as dark areas in 3D ultrasound images. The inner body fluids can be blood fluid or a fluid-containing organ, such as a bladder or a gallbladder. One target of the present invention is to detect medical conditions associated with internal bleeding. In another aspect, the present invention uses the position of the detected internal organs to decide whether the detected fluid area is an inner body fluid-containing organ or if the fluid area is due to internal bleeding.

It is important to note that ultrasound volumetric images suffer several problems including: high levels of speckle noise, contrast variation among volume data, and discontinuities among internal organ body boundaries. To reduce the abovementioned ultrasound 3D imaging problems, the present invention combines two methods, noise reduction using the double-sparsity dictionary learning approach and contrast enhancement using the Mardia and Hainsworth method.

It should be noted that the double-sparsity dictionary learning method is used to train a sparse-dictionary that best describes the valuable signal and is not representative for the speckle noise. Thus, the reconstructed image using the generated dictionary in the sparse representation framework is de-speckled.

It should further be noted that the Mardia and Hainsworth method is a local thresholding approach that localizes the classification of points into tissue and non-tissue regions. To improve the contrast of the ultrasound volumetric data, the Mardia and Hainsworth approach is applied to the de-speckled volume to create a binary volume which has a value of one at tissue voxels and zero at non-tissue voxels.

Then, the weighted summation of the de-speckled volume and the binary volume provides a De-Speckled Contrast Enhanced (DSCE) volume at the preprocessing step.

In another aspect, the present invention provides an effective way for a user to manually select initial seed points or to manually draw initial contours for the segmentation method. Specifying multiple initial seeds provides flexibility to improve segmentation results. In addition to the capability of manually selecting initial points, the present invention may automatically offer seed points in the ultrasound volume. The model of human visual attention system is used to select conspicuity points in the ultrasound volume. The user can switch between manually selected points/contours or automatically specified points.

Two segmentation strategies are preferably applied in the present invention: (1) a deformable model based on prior shapes to detect organs, and (2) a combination of active contour model, Level-Set, and region growing method to detect fluid areas. The first strategy is applied to detect an organ, such as a kidney or liver, based on the prior knowledge of its shape. The known prior shape is defined as a zero Level-Set function and a deformable model is used to fit the shape to a region in the volume. The deformable model consists of global and local transforms. The global transform is formed by rotation, translation and a single scale factor with 7 parameters. The global transform finds a possible location of the prior shape in the volume. The local transform is then applied using the Level-Set approach with several iterations to maintain the minimal changes in the prior shape. The deformed shape is then analysed to determine how well it fits in the volume in order to decide whether the object has been detected or not. This task is performed for prior shapes of organs in an online fashion for the stream of volumetric data.

In ultrasound volumetric data, the fluid areas appear as wide homogenous dark regions. The initial seed points, either manually drawn or automatically determined, are used to initiate the segmentation of the fluid regions. The segmentation processing step is a hybrid approach that consists of the 3D Snake active contour model, 3D Level-Set, and the minimum variance region growing approach. The 3D Snake model is robust against discontinuities among object boundaries, while the 3D Level-Set approach provides better accuracy.

The present invention takes advantage of the benefits of both approaches, including robustness against discontinuities and the high accuracy of segmentation. The 3D Snake is initiated with a sphere and the sphere is iteratively evolved to extract the object boundary. The segmentation result of the 3D Snake is a 3D surface that is used to initiate the Level-Set function. The 3D Snake surface is first converted to a binary volume with ones inside the surface and zeroes outside the surface. The 3D image-filling operator is applied to fill the inside of the surface. For this, a surface without any gap is required. However, the 3D Snake points are discretely separated and an interpolation is required to create a closed surface. This is achieved by using a few iterations of the region growing approach. Then, the image-filled morphological operator provides a filled binary volume which can be easily used to create the initial Level-Set function. Further iterations of the Level-Set deformation can be used to improve the segmentation accuracy.

The preprocessing step is added to improve the accuracy of the segmentation task. The two purposes of the preprocessing step are to reduce the amount of the speckle noise and to improve the contrast of the volumetric data. The sparse representation framework is employed to reduce the amount of speckle noise in the volumetric ultrasound data. Also, the volume intensities in tissue voxels are improved by adding a weighted volumetric classification result obtained using the Mardia and Hainsworth approach.

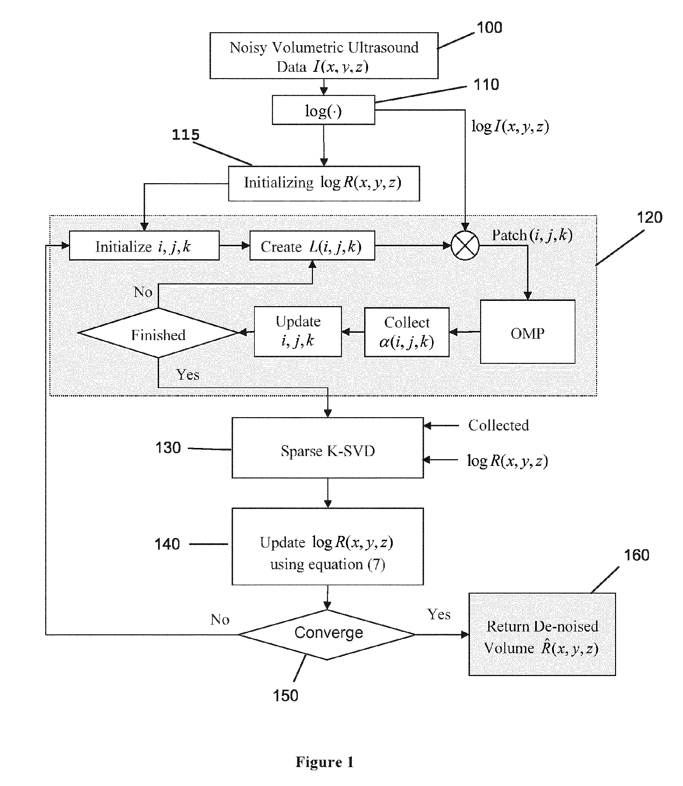

A block diagram of a denoising approach based on a sparse K-SVD (K-Singular Value Decomposition) is shown in Figure 1.

Referring to Figure 1, the input data of the present invention is the 3D ultrasound images, also known as the 3D ultrasound volume. In this process, the first step 100 is that of determining the volumetric data $I(x, y, z)$, in which $x = [1,..., S_x]$, $y = [1,..., S_y]$ and $z = [1,..., S_z]$. Each $I(x, y, z)$ is a volumetric pixel (voxel) with intensity levels in $I(x, y, z) \in [0,1,2,..., 255]$. With the current ultrasound imaging technology, the ultrasound volume data has a high level of multiplicative noise, also called the speckle noise. The general formulation for the speckle noise is represented as,

$$I(x, y, z) = \varepsilon_m(x, y, z) \times R(x, y, z) + \varepsilon_a(x, y, z) \qquad (1)$$

where $\varepsilon_m(x, y, z)$ and $\varepsilon_a(x, y, z)$ are multiplicative and additive noise components, respectively. $R(x, y, z)$ is the actual data. The additive noise component is negligible and can be remove from the above formula. In addition to the speckle noise, the intensity level of the ultrasound volumetric data varies in different locations in the 3D volume. Therefore, fitting a global probability mixture distribution model is subject to inaccuracy in ultrasound volumetric data.

De-Speckling using Sparse K-SVD

Consider 3D patches of size $\sqrt[3]{n} \times \sqrt[3]{n} \times \sqrt[3]{n}$ taken from the volumetric ultrasound data and reordered into a column vector, $x_i \in R^n$. Having a dictionary, $D \in R^{n \times m}$, of $m$ atoms, $d, \in R^n$, the sparse representation of $x_i$ is formulated as follows,

$$\hat{\alpha} = \arg \min_{\alpha} \|\alpha\|_0 \quad subject\ to \quad \|x - D\alpha\|_2^2 < \varepsilon \qquad (2)$$

where $\alpha$ is a sparse vector with a few number of non-zero coefficients. The dictionary, D, can be an analytical dictionary. If we consider the noise to have a normal distribution with zero-mean and unit variance, the MAP (maximum a posteriori probability) solution of the denoised signal is attained by solving equation (2) and finding $\hat{x}_i = D\hat{\alpha}$. Applying a Lagrange multiplier, equation *(2)* becomes,

$$\hat{\alpha} = \arg \min_{\alpha} \|x - D\alpha\|_2^2 + \mu \|\alpha\|_0 \qquad (3)$$

The solution to equation (3) is achieved using the Orthogonal Matching Pursuit (OMP) method. The sparse representation task can be better performed by using learned-based dictionaries. In order to denoise the ultrasound volume, $I(x, y, z)$, aiming to achieve the noise-free volume, $R(x, y, z)$, the second step 110 (from Figure 1) is to apply the logarithm transform on equation *(1)* to convert the multiplication operator into the summation operator as follows,

$$\log(I(x, y, z)) = \log(\varepsilon_m(x, y, z)) + \log(R(x, y, z)) \qquad (4)$$

Note that the $\varepsilon_a(x, y, z)$ is removed as a negligible term in the equation *(1)* (see step 115 in Figure 1). Our desired output is to find the noise-free data which is R(x, y, z) . Considering the whole volume to be denoised, equation (3) is reformulated as,

$$\arg\min_{\{R(x,y,z),\alpha_{i,j,k},D\}} \left( \left( \sum_{i,j,k} \left\| L_{i,j,k} \times \log R(x,y,z) - D\alpha_{i,j,k} \right\|_2^2 + \mu_{i,j,k} \left\| \alpha_{i,j,k} \right\|_0 \right) \\ + \lambda \left\| \log R(x,y,z) - \log I(x,y,z) \right\|_2^2 \right) \quad (5)$$

$L_{i,j,k}$ is an operation that extracts a 3D patch from the volumetric data. $\lambda$ is the multiplier which controls the proximity of the logarithm of the input noisy-image, $\log I(x, y, z)$, with the logarithm of the noise-free data, $\log R(x, y, z)$, in the entire volume. The analytical or fixed dictionaries can represent signals based on specific characteristics, whereas learned-based dictionaries improve representation accuracy sparsity level. A method developed by M. Aheron, M. Elad, and A. Bruckstein, called the sparse K-SVD method, considers the learned dictionary to be itself a sparse representation of a fixed dictionary (such as DCT). This method makes a bridge between analytical or fixed dictionaries and learned-based dictionaries to take advantages of both dictionaries. This method has been shown to be more effective for image denoising applications. In this case, the sparse dictionary is defined as $D = \Phi A$ where $\Phi$ is a DCT dictionary and A is the matrix of sparse coefficients. In other words, each dictionary atom, $d_i$, has a sparse vector $A_i$ to represent it based on the fixed dictionary, $\Phi$. The sparse-dictionary is applied in equation (5) and the modified formula is obtained as follows,

$$\arg\min_{\{R(x,y,z),\alpha_{i,j,k},D\}} \left( \left( \sum_{i,j,k} \left\| L_{i,j,k} \times \log R(x,y,z) - \Phi A\alpha_{i,j,k} \right\|_2^2 + \mu_{i,j,k} \left\| \alpha_{i,j,k} \right\|_0 \right) \\ + \lambda \left\| \log R(x,y,z) - \log I(x,y,z) \right\|_2^2 \right) \quad (6)$$

An iterative procedure is applied to minimize equation *(6)* in which three individual steps are performed in each iteration. Having the initialization of *log R(x, y, z)* = log *I(x, y, z)*, the first part of the iterative procedure calculates $\alpha_{i,j,k}$ by fixing D and log *R(x, y, z)*. This part of the procedure is separately performed for each $\alpha_{i,j,k}$, related to a specific 3D patch, using the OMP method (see step 120). In the next step of the denoising method, step 130, the dictionary, D, is updated using the sparse K-SVD approach by fixing $\alpha_{i,j,k}$ and log *R(x, y, z)*. Afterwards, in step 140, the minimization problem in equation *(6)* is optimized with respect to log *R(x, y, z)*. The optimization solution is obtained using the following formula,

$$\log R(x,y,z) = \left( \lambda I_{id} + \sum_{ijk} L_{i,j,k}^T L_{i,j,k} \right)^{-1} \left( \lambda I(x,y,z) + \sum_{ijk} L_{i,j,k}^T \Phi A\alpha_{i,j,k} \right) \quad (7)$$

where $I_{id}$ is an identity matrix. The first matrix on which an inverse operation is applied is a diagonal matrix. Therefore, the equation *(7)* can be separately performed for each 3D patch in the volume. After the optimization, the updated log R(x, y, z) is checked (step 150) with the previous value to decide whether to proceed with another iteration, or to stop the denoising procedure. This process results in step 160, the return of the estimation of the noise-free volumetric data, $\hat{R}(x, y, z)$.

Enhancing Tissue Voxels' Intensity

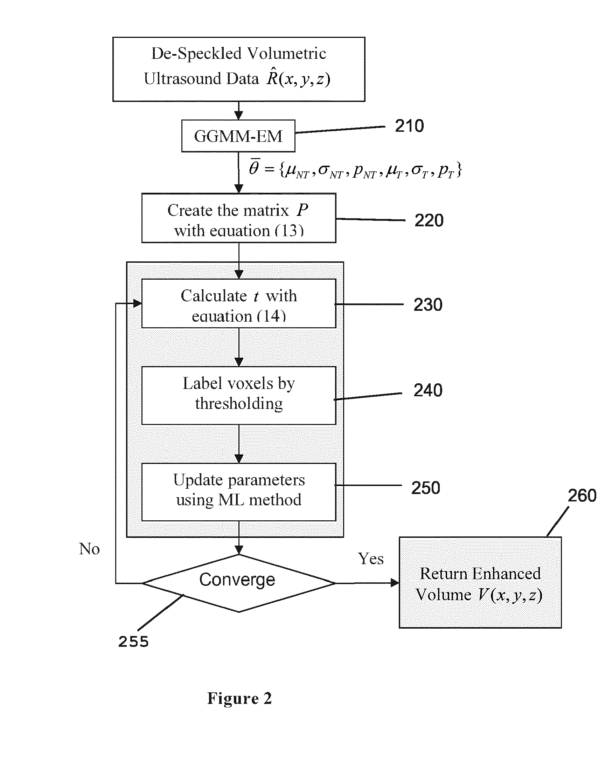

A block diagram of the procedure for the tissue voxels' intensity enhancement is illustrated in Figure 2.
As discussed previously, ultrasound volumetric data suffers from inconsistent intensity level in object boundaries. More specifically, a particular object may have brighter boundaries in some parts of its surface and darker boundaries in other parts. This problem highly affects the proper functioning of the segmentation process. A new approach is presented herein to equalize tissue voxels' intensities using the following equation,

$$V(x,y,z) = \alpha B(x,y,z) + (1-\alpha)\hat{R}(x,y,z) \quad (8)$$

where $\alpha$ is the mixing factor and $B(x, y, z)$ is volumetric data,

$$B(x, y, z) = \begin{cases} \mu_T & if \quad (x, y, z) \quad is \quad tissue \\ \mu_{NT} & if \quad (x, y, z) \quad is \quad non-tissue \end{cases} \qquad (9)$$

where $\mu_T$ and $\mu_{NT}$ are mean values of the tissue and non-tissue voxels, respectively. To find $\mu_T$ and $\mu_{NT}$, the expectation maximization (EM) method is applied to find parameters of the Gaussian-Gaussian Mixture Model (GGMM) to fit it on the input volumetric data.

The Mardia and Hainsworth approach is used to determine tissue and non-tissue voxels in the de-speckled ultrasound volumetric data, $\hat{R}(x, y, z)$. In the Mardia and Hainsworth approach, voxels in a local neighbourhood are considered an isotropic random Gaussian process, where the cross-covariance of two voxels are dependent on their Euclidean distance as follows,

$$E[\hat{R}(x, y, z)] = \mu(x, y, z), Cov[\hat{R}(x, y, z), \hat{R}(x_N, y_N, z_N)] = \sigma^2(x, y, z)\rho(\|(x, y, z) - (x_N, y_N, z_N)\|_2) \qquad (10)$$

where $\rho(dist)$ is an isotropic correlation function in which $\rho(0) = 1$. The correlation function may be selected as,

$$\rho(d) = \exp\{-(d)^\eta / \varphi\} \qquad (11)$$

where $\eta$ and $\varphi$ are two parameters that control the decaying of the correlation function. This definition maintains a smooth decay of the correlation function by increasing the distance between a voxel $(x, y, z)$ and its neighbour $(x_N, y_N, z_N)$. The linear combination of neighbour voxels is calculated to estimate the voxel $(x, y, z)$ as $\tilde{R}(x, y, z) = \sum_{n \in Neighbors(x,y,z)} \gamma_n \hat{R}(x_n, y_n, z_n)$. The assumption of $\sum \gamma_n = 1$ provides $\tilde{\mu}_T = \mu_T$, $\tilde{\mu}_{NT} = \mu_{NT}$. Accounting for variances provides,

$$\tilde{P} = \vec{\gamma}^T P \vec{\gamma}, \quad \tilde{\sigma}_T^2 = \tilde{P}\sigma_T^2, \quad \tilde{\sigma}_{NT}^2 = \tilde{P}\sigma_{NT}^2 \qquad (12)$$

where $\bar{\gamma} = [\gamma_1, \gamma_2, \gamma_3, ..., \gamma_N]^T$ is the vector of weights. $\sigma_T$ and $\sigma_{NT}$ are standard deviations of tissue and non-tissue voxels obtained using the GGMM-EM method. P is the correlation function matrix defined as follows,

$$P = \begin{bmatrix} \rho(0) & \rho(1) & \rho(1) & \cdots \\ \rho(1) & \rho(0) & \rho(1) & \cdots \\ \rho(2) & \rho(1) & \rho(0) & \cdots \\ \vdots & \vdots & \vdots & \ddots \end{bmatrix}_{N \times N} \qquad (13)$$

Having prior probabilities of tissue voxels, $p_T$, and non-tissue voxels, $p_{NT}$, the spatial thresholding is obtained by modifying the Lloyd spatial thresholding equation as follows,

$$t = \frac{(\mu_T \sigma_{NT}^2 - \mu_{NT}\sigma_T^2)}{(\sigma_{NT}^2 - \sigma_T^2)} \pm \frac{(\sigma_{NT}\sigma_T)}{(\sigma_{NT}^2 - \sigma_T^2)}\{(\mu_{NT} - \mu_T)^2 + 2\tilde{P}(\sigma_{NT}^2 - \sigma_T^2)\log((p_T\sigma_{NT})/(p_{NT}\sigma_T))\}^{0.5} \qquad (14)$$

The local mean thresholding method is then applied to label voxels into tissue and non-tissue classes as follows,

$$\begin{cases} \tilde{R}(x,y,z) \geq t & label : Tissue \\ \tilde{R}(x,y,z) < t & label : non-Tissue \end{cases} \qquad (15)$$

After labelling all voxels, the estimation of parameters $\overline{\theta} = \{\mu_{NT}, \sigma_{NT}, p_{NT}, \mu_T, \sigma_T, p_T\}$, is updated with Maximum Likelihood (ML) method. If convergence is not attained, the process is repeated with equation (14), unless the volume intensity enhancement process is finished and the enhanced volume, $V(x, y, z)$, is obtained using the equation (8). $V(x, y, z)$ is now ready to be used by the segmentation procedure.

From Figure 2, it can be seen that the intensity enhancement procedure has a number of steps. The first step 200 is that of determining the de-speckled ultrasound data. This data is the result of the procedure illustrated in Figure 1. Afterwards, in step 210, $\mu_T$ and $\mu_{NT}$ are found by applying the the expectation maximization (EM) method to find parameters of the Gaussian-Gaussian Mixture Model (GGMM). The matrix P is then found using equation (13) above (step 220). The value for spatial thresholding, $t$ is then calculated using equation (14) (step 230). The relevant voxels are then labelled (step 240) and, using Maximum Likelihood, the parameters $\overline{\theta} = \{\mu_{NT}, \sigma_{NT}, p_{NT}, \mu_T, \sigma_T, p_T\}$ are updated (step 250). A check is then made to determine if the values are converging (step 255). If there is no convergence, the procedure returns to step 230 for another iteration. If there is convergence, the enhanced volume data is returned as the procedure's output (step 260).

<u>Initial Points and Surface Selection</u>

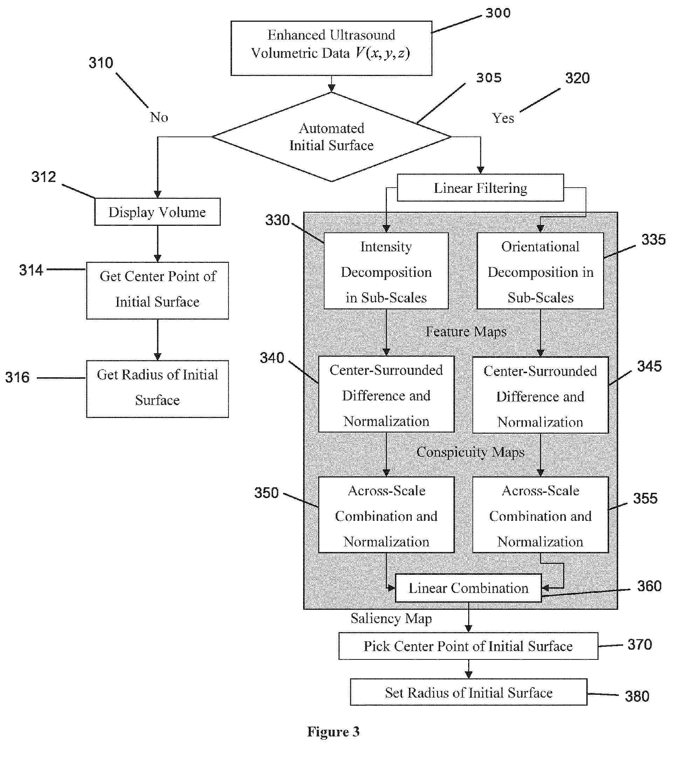

A block diagram of the initial surface selection procedure is shown in Figure 3.

The segmentation task using the 3D Snake model requires an initial surface to start the process. There are two methods to initiate the 3D Snake method: (1) manual selection of an initial surface, and (2) automatic selection of initial surface based on human visual attention system. The human visual attention system models the manual selection of points in the ultrasound volumes.

The initial surface selection procedure in Figure 3 starts with the intensity enhanced data that was the output from the procedure in Figure 2 (step 300). A determination is then made as to whether the initial surface selection is to be made manually or automatically (step 305). If the selection is to be performed manually (step 310), then the left sequence in Figure 3 is followed. For an automatic selection (step 320), the right sequence in Figure 3 is followed.

In the manual selection of the initial surface, the user displays the volume (step 312) by moving among the volume in different planes and deciding where to insert the initial point (step 314) by clicking on the mouse button. Then, the user starts to draw a sphere by moving the cursor to determine the radius of the sphere (step 316). The manually drawn sphere is finalized by clicking on the mouse button. The outputs of the manually surface selection task are the center point, $C_{surf} = [x_c, y_c, z_c]^T$, and the surface radius, $r_{surf}$.

In the automatic selection of the initial surface (step 320), the human visual attention system model finds some points inside the ultrasound volume to alternatively select the center point of the initial surface. The first step of the human visual system is to decompose the input visual field into topographical maps. This is performed for the enhanced ultrasound volume. $V(x, y, z)$ is decomposed to multi-scale intensity (step 330) and orientation-based (step 335) maps, also called topographic maps. The topographic intensity maps are labeled with $V(x, y, z, \sigma) \in R^{Mx(\sigma) \times My(\sigma) \times Mz(\sigma)}$ where $M_x(\sigma) = S_x/2^\sigma$, $M_y(\sigma) = S_y/2^\sigma$ and $M_z(\sigma) = S_z/2^\sigma$. Intensity-based topographic maps are generated for $\sigma \in \{0,1, 2, 3, 4, 5, 6, 7,8\}$, resulting in 9 different scales. The orientation-based topographic maps are generated for 4 directions of $\theta \in \{0, \pi/4, \pi/2, 3\pi/4\}$. Thus, there exist 36 multi-scale orientation-based topographic maps, $O(x, y, z, \sigma, \theta)$.

Feature maps are then generated from topographic maps (steps 340, 345). This reflects the lateral inhibition property of the nervous system which causes the neighbours to influence each other. Thus, only those points in topographic maps which significantly differ from their surroundings survive in the feature maps. To generate this feature map, the fine resolution topographic map is subtracted with its low-passed version obtained by up-sampling of coarser scales maps. This is formulated as

$$V(x, y, z, \sigma', \delta) = V(x, y, z, \sigma')(-)V(x, y, z, \sigma' + \delta) \qquad (16)$$

where $\sigma' \in \{2, 3, 4\}$ and $\delta \in \{3,4\}$. The (-) operation subtracts the left side operand in $\sigma'$ scale with the up-sampled version of the right operand from $\sigma' + \delta$ scale to $\sigma'$ scale. The result is 6 feature maps related to the image intensity property. The same operation is applied on orientation-based topographic maps, but in different directions as follows,

$$O(x, y, z, \sigma', \delta, \theta) = O(x, y, z, \sigma', \theta)(-)O(x, y, z, \sigma'+\delta, \theta) \qquad (17)$$

The orientation-based features are 24 maps, created in 4 directions and 6 scales' combinations. These steps provide 30 feature maps in total.

After the feature maps are created, conspicuity maps are created by summing together feature maps in the scale $\sigma = 4$. In step 350, 355, the intensity based features are summed to arrive at a single intensity-based conspicuity map, according to the following summation formula,

$$V_c(x, y, z) = N(V(x, y, z, 2, 5))(+)N(V(x, y, z, 2, 6))(+)...(+)N(V(x, y, z, 4, 8)) \qquad (18)$$

where N is the normalization operation. The purpose of using this normalization operation is to reduce the influence of the noisy data in a particular feature map and to increase the importance of stand-alone pixels in other feature maps. The result of steps 350, 355 is five conspicuity maps including one intensity-based map and four orientation-based conspicuity maps in different directions as follows,

$$O_c(x, y, z, \theta) = N(O(x, y, z, 2, 5, \theta))(+)N(O(x, y, z, 2, 6, \theta))(+)...(+)N(O(x, y, z, 4, 8, \theta)). \qquad (19)$$

Step 360 is that of summing the normalized versions of the conspicuity maps to produce the Saliency map, $S(x, y, z)$. Having this saliency map, the visual attention point refers to the location with the maximum value. From the saliency map, the center point, $\overline{C}_{surf} = [x_c, y_c, z_c]^T$, is selected (step 370) and the surface radius, $r_{surf} = R_{default}$ is set (step 380). The $R_{default}$ can be any arbitrary value and is preferably 5.

Volumetric Ultrasound Data Segmentation

In the present invention, the segmentation task consists of two different strategies using: (1) deformable model based on prior shapes to detect organs, such as kidneys and livers, and (2) a combination of active contour model, Level-Set and region growing methods to detect fluid areas. Provided below are the mathematical details of these strategies. In a preferred embodiment of the present invention, the ultrasound data is used to detect the location of the kidney or the liver. As such, the present disclosure will focus on analysis for the detection of kidneys and livers. However, it will be understood by a person skilled in the art that a similar analysis could be used to detect the location of any internal organ.

In the first strategy, in which a deformable model based on prior shapes is used to detect organs, the location of the ultrasound-probe is not known to the system. Thus, the kidney and liver detection strategy is applied to locate the ultrasound-probe. This provides a possible way to discriminate between fluid-carrying organs and free fluids in volumetric data. One assumption is that kidney and liver have standard shapes and can be detected based on their shape in the volumetric data. Based on this assumption, the prior shapes of kidney and liver are deformed to find possible fits in each volume in the volumetric data. The deformation is performed in both global and local fashions. The global deformation is applied to fit the location and orientation of the shape in the volume while the local transformation is applied to slightly change the shape to maximize the fit of the shape in a possible location in the volume.

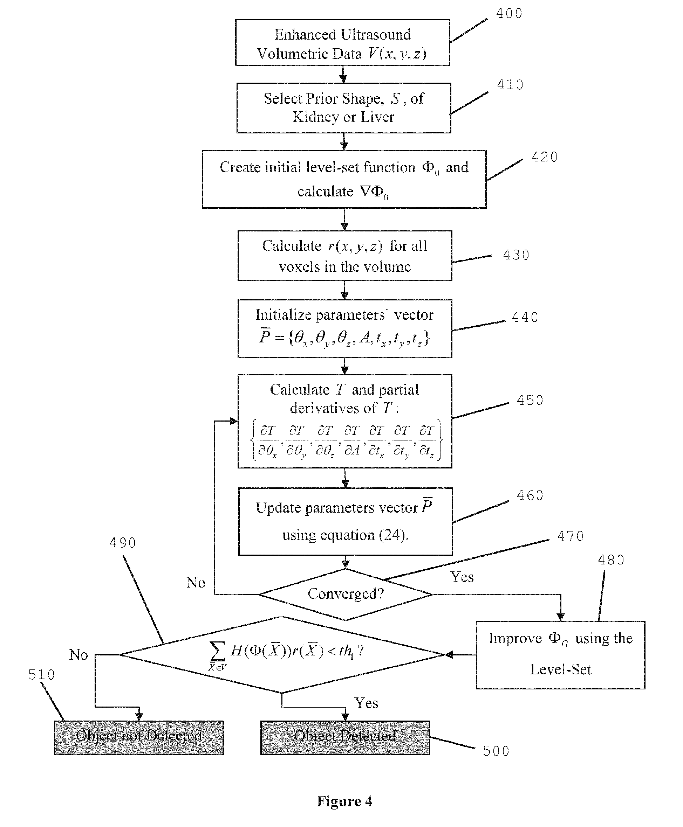

Referring to Figure 4, the steps in a procedure for detecting the liver or kidney are illustrated. The procedure begins with the enhanced ultrasound volumetric data which was the output of the procedure from Figure 2 (step 400). A prior shape, $S$, is first selected (step 410). For this procedure, the prior shape, $S$, is defined as the zero Level-Set function. Thus, the Level-Set function is defined as $\{\Phi(\overline{X}) \mid \overline{X} = [x, y, z]^T\}$ in which $\Phi^{-1}(0)=S$, $\Phi(\overline{X}_{out}) < 0$ and $\Phi(\overline{X}_{in}) > 0$, where $\overline{X}_{out}$ and $\overline{X}_{in}$ refer to voxels outside and inside the prior shape, respectively. An initial level-set function $\Phi_0$ is then created (step 420). To find the best fit of prior shapes in the volume, the following error function is minimized,

$$\min_{\Phi} \sum_{\overline{X} \in V} H(\Phi(\overline{X}))r(\overline{X}) \qquad (20)$$

where $H(x)$ is the Heaviside function and is positive for $x > 0$ and negative elsewhere. $r(\overline{X}) = r_T(\overline{X}) - r_{NT}(\overline{X})$ is based on intensities of voxels and the distribution model obtained in the enhancing tissue voxels' intensity procedure as follows,

$$r_T(\bar{X}) = -(V(\bar{X}) - \mu_T)^2 / (2\sigma_T^2) + \log(p_T / (\sqrt{2\pi}\sigma_T)) \qquad (21)$$

$$r_{NT}(\bar{X}) = -(V(\bar{X}) - \mu_{NT})^2 / (2\sigma_{NT}^2) + \log(p_{NT} / (\sqrt{2\pi}\sigma_{NT}))$$

Values for $r(x,y,z)$ for all voxels in the volume can then be calculated (step 430).

The Level-Set function is then written as the deformation of the prior Level-Set $\Phi_0$,

$$\Phi(\bar{X}) = \Phi_0(T \cdot \bar{X}') \qquad (22)$$

where $\bar{X}' = [\bar{X}^T, 1]^T$ and $T$ is a global transformation in the homogenous geometry and is defined with 7 parameters as follows,

$$T = T_1 \times T_2 \times T_3$$

$$T_1 = \begin{bmatrix} 1 & 0 & 0 & c_x + t_x \\ 0 & 1 & 0 & c_y + t_y \\ 0 & 0 & 1 & c_z + t_z \\ 0 & 0 & 0 & 1 \end{bmatrix} \qquad (23)$$

$$T_2 = \begin{bmatrix} A\cos(\theta_y)\cos(\theta_z) & \{\sin(\theta_x)\sin(\theta_y)\cos(\theta_z) - \cos(\theta_x)\sin(\theta_z)\} & \{\cos(\theta_x)\sin(\theta_y) + \sin(\theta_x)\sin(\theta_z) \\ \cos(\theta_y)\sin(\theta_z) & \{A(\sin(\theta_x)\sin(\theta_y)\sin(\theta_z) + \cos(\theta_x)\cos(\theta_z))\} & \{\cos(\theta_x)\sin(\theta_y) - \sin(\theta_x)\cos(\theta_z \\ -\sin(\theta_z) & \cos(\theta_y)\sin(\theta_x) & A\cos(\theta_x)\cos \\ 0 & 0 & 0 \end{bmatrix}$$

$$T_3 = \begin{bmatrix} 1 & 0 & 0 & -c_x \\ 0 & 1 & 0 & -c_y \\ 0 & 0 & 1 & -c_z \\ 0 & 0 & 0 & 1 \end{bmatrix}$$

where $[c_x, c_y, c_z]^T$ is the center of the prior shape, and $\bar{P} = \{\theta_x, \theta_y, \theta_z, A, t_x, t_y, t_z\}$ are 7 parameters of the global transformation. These parameters can thus be initialized (step 440). An iterative update procedure (steps 450-470) is applied to modify these parameters to fit the shape in the volume. The initial set for these values is {0,0,0,1,0,0,0}. For this iterative procedure, $T$ and partial derivatives of T are calculated (step 450). The following equation (using the results of step 450) is applied to iteratively modify these parameters (step 460),

$$P_i^{iter+1} = P_i^{iter} + K_1 \sum_{X \in V} r(T^{-1} \times X') \times <\nabla\Phi_0, \frac{\partial(T(\bar{P}))}{\partial P_i} \times (T^{-1} \times X')> \qquad (24)$$

where $K_1$ is a constant number that controls the updating step size.

The iterative process is repeated until a convergence is attained. A check (step 470) determines if convergence has been attained or not. If there is no convergence in the results, then the logic flow returns to step 450 for another iteration. If there is convergence, the result is a final Level-Set function, $\Phi_G$, that can be used by the Level-Set method (step 480) to improve the fitness accuracy in the volume. A test is then performed to determine to see if

$$\sum_{\bar{X} \in V} H(\Phi_G(\bar{X}))r(\bar{X})$$

490 is less than a constant threshold value, $th_1$ (decision 490). If the result of the test is positive, then the object sought (a kidney or liver) has been detected (step 500). If the result is negative, then the object sought has not been detected (step 510).

In the second strategy used to detect internal fluids, the 3D Snake approach provides a highly robust method against boundaries' discontinuities, if its parameters are correctly adjusted. However, the precise segmentation using the 3D Snake method requires an extensive number of sample points, resulting in a very high computational cost. On the other hand, the Level-Set approach provides accurate segmentation with less computational cost. However, this approach is not resistant against discontinuities among object boundaries.

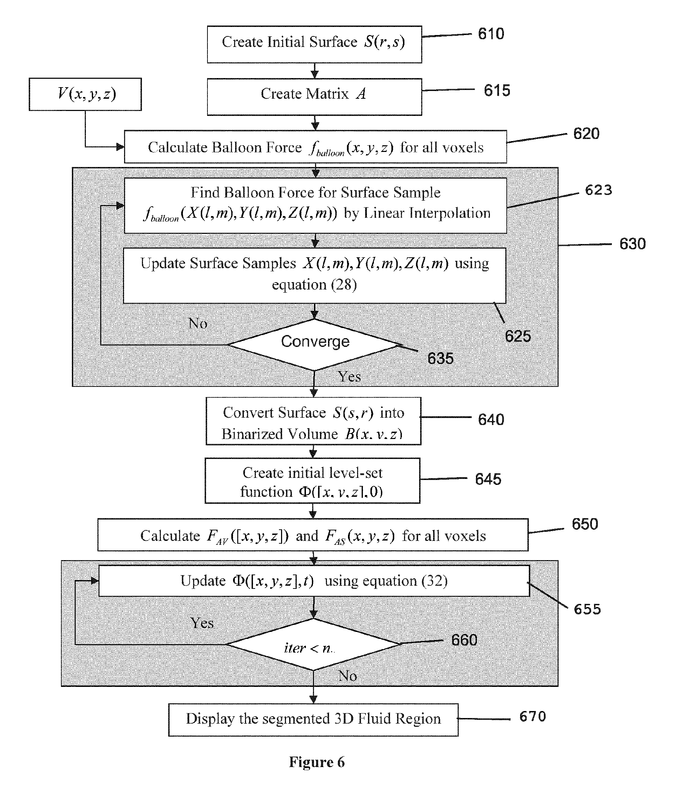

In another aspect of the present invention, a novel approach is to make a bridge between the 3D Snake and Level-Set methods. This combined approach provides the robustness of the 3D Snake method as well as the accuracy of Level-Set method. The steps in this combined approach are detailed in Figure 6.

As can be seen in Figure 6, in the 3D Snake method, a surface is represented with two parameters, $s \in [0,1]$ and $r \in [0,1]$, as $S(s, r) = \{X(s, r), Y(s, r), Z(r, s)\}$. The manual or automatic initial surface that was determined as noted above is used to define the initial surface $S(s,r)$ (step 610). For this implementation of the invention, it is assumed that the surface follows the boundary condition, $S(0, r) = S(1, r)$, and is initialized as a sphere. The surface evolution to segment the region of interest is formulated as the following energy function,

$$E_{\text{int}} = \int \alpha_s \left|\frac{\partial S}{\partial s}\right|^2 + \alpha_r \left|\frac{\partial S}{\partial r}\right|^2 + \beta_s \left|\frac{\partial^2 S}{\partial s^2}\right|^2 + \beta_r \left|\frac{\partial^2 S}{\partial r^2}\right|^2 + \beta_{sr} \left|\frac{\partial^2 S}{\partial s \partial r}\right|^2 + E_{volume}(S(s,r))dsdr \qquad (25)$$

where $\alpha_s$ and $\alpha_r$ are two parameters that control the surface tension along s-axis and r-axis, respectively. $\beta_s$ and $\beta_r$ determine the surface rigidity along s-axis and r-axis, respectively. Also, $\beta_{sr}$ prevents the surface against twisting. To simplify the problem, one can assume that $\alpha = \alpha_s = \alpha_r$ and $\beta = \beta_s = \beta_r = \beta_{sr}$. This assumption provides an easier parameter adjustment. The Euler-Lagrange solution to the equation is,

$$\alpha \frac{\partial^2 S}{\partial s^2} + \alpha \frac{\partial^2 S}{\partial r^2} + \beta \frac{\partial^4 S}{\partial s^4} + \beta \frac{\partial^4 S}{\partial r^4} + \beta \frac{\partial^4 S}{\partial s^2 \partial r^2} + \nabla E_{volume}(S(s,r)) = 0 \qquad (26)$$

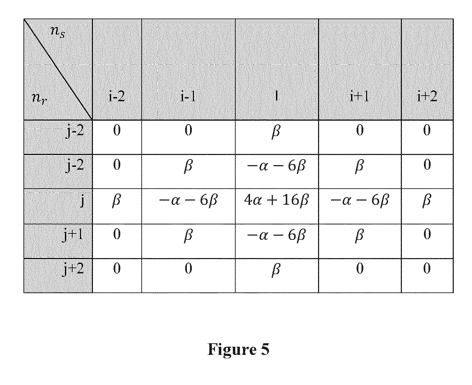

To numerically solve the Euler-Lagrange equation, the surface model is discretized to $S(n_s, n_r)$ where $n_s \in \{1,2,..., N_s\}$ and $n_r \in \{1, 2,..., N_r\}$. This conversion maps $s = 0$, $s=1$, $r=0$ and $r = 1$ into $n_s = 1$, $n_s = N_s$, $n_r = 1$ and $n_r = N_r$, respectively. After simplifying the difference equation obtained by discretizing equation *(26)*, the relation of each single surface sample, $S(i, j)$, and its neighbour samples is achieved, as shown in Figure 5. The surface samples are reshaped from the 2D matrix form, $S \in R^{N_s \times N_r}$, into a vector form, $S'(k) = [X(l, m), Y(l, m), Z(l, m)]$ where $l = [k / N_r]$ and $m = k - l \times N_r$. Additionally, the balloon force is added to let the surface evolve. The image force bounds the evolving surface to stop in desired boundaries. At each surface sample point, the balloon force is normal to the surface and inflates the surface outward. The difference equation *(26)* is converted into the following matrix representation form,

$$A \times X + \frac{\partial V(S)}{\partial x} + \gamma f_{balloon}^x(S) = 0 \qquad (27)$$

$$A \times Y + \frac{\partial V(S)}{\partial x} + \gamma f_{balloon}^y(S) = 0$$

$$A \times Z + \frac{\partial V(S)}{\partial x} + \gamma f_{balloon}^z(S) = 0$$

where the matrix $A \in R^{(N_s \times N_r) \times (N_s \times N_r)}$ represents the internal force, and $f_{balloon}^x(S)$, $f_{balloon}^y(S)$ and $f_{balloon}^z(S)$ are projections of $f_{balloon}$ on the x-axis, y-axis and z-axis, respectively. For the next step in the procedure in Figure 6, this matrix A is created (step 615). After this, the balloon force $f_{balloon}(x,y,z)$ is calculated for all the voxels (step 620) using as input the enhanced ultrasound volumetric data $V(x,y,z)$. Equation (27) must, therefore, now be solved. The iterative solution to the equation (27) is

$$X_t = (A+I)^{-1}(X_{t-1} - \frac{\partial V(S)}{\partial x} - \gamma f_{balloon}^x(S)) \qquad (28)$$

$$Y_t = (A+I)^{-1}(Y_{t-1} - \frac{\partial V(S)}{\partial y} - \gamma f_{balloon}^y(S))$$

$$Z_t = (A+I)^{-1}(Z_{t-1} - \frac{\partial V(S)}{\partial z} - \gamma f_{balloon}^z(S))$$

where $t$ is the iteration number and $I \in R^{(N_s \times N_r) \times (N_s \times N_r)}$ is the unit matrix. As $S$, are non-integer values, each value of $V(S_t)$ should be interpolated from the level of the surrounding 3D image voxels. The evolution of the surface is an iterative process (box 630) performed by repeating the equation (28). The evolution stops when the 3D Snake surface finds the object boundaries in the volume. In this iterative process, step 623 is that of finding the balloon force for the various surface samples by linear interpolation. The surface samples are then updated using equation (28) (step 625). A check is then made (step 635) to determine if the results are converging. If there is no convergence in the results, the logic flow returns to step 623 for another iteration. If there is convergence, then the procedure moves to the next step.

After the 3D Snake approximately finds the object boundaries, the 3D surface is converted into an initial Level-Set function. To achieve the initial Level-Set function, the surface samples are placed in a binarized volume (step 640). Every four neighbour points are interpolated to create a continuous surface in the binarized volume with values equal to 1. The surface is turned into a shape in the 3D volume. Using the image-fill morphological operation, the shape can be filled by values equal to 1. Then, the initial 3D Level-Set function, $\Phi_0$, is created (step 645) by making voxels on the shape surface equal to zero, making voxels inside the shape equal to 1, and making voxels outside the shape equal to -1. The zero-Level-Set function is pointing to the resultant 3D Snake surface, $\Phi_0(S) = 0$.

The Level-Set function $\Phi([x, y, z], t)$, assigns a level to each point, $[x, y, z]$, in the 3D grid and $\Phi([x, y, z], 0) = \Phi_0$. The volume containing points with non-negative Level-Set values are called the Front. The Level-Set function iteratively updates along its normal vector fields by $F([x, y, z])$, which controls the speed of Level-Set change at each time-point. The Level-Set function stops changing at boundaries. The speed function, $F([x, y, z])$, has two components, $F([x, y, z]) = F_A([x, y, z]) + F_G([x, y, z])$, where $F_A([x, y, z])$ is similar to the balloon force and $F_G([x, y, z])$ maintains the shape's smoothness. $F_A([x, y, z])$ is divided into $F_{AV}([x, y, z])$ and $F_{AS}([x, y, z])$. $F_{AV}([x, y, z])$ is the normal speed vector to the Level-Set function at each point, while $F_{AS}([x, y, z])$ consists of vectors pointing toward image edges. These values are then calculated for each of the voxels (step 650). Because the smoothness is provided by the 3D Snake, $F_G([x, y, z])$ is eliminated and the evolution formula is driven as follows,

$$\frac{\partial \Phi([x,y,z],t)}{\partial t} + F_{AV}([x,y,z]) \cdot \nabla \Phi([x,y,z],t) + F_{AS}([x,y,z]) \cdot \nabla \Phi([x,y,z],t) = 0 \qquad (29)$$

The normal term of the first speed term is only effective, and therefore the above formula is again simplified as,

$$\frac{\partial \Phi([x,y,z],t)}{\partial t} + F_{AV}([x,y,z]) |\nabla \Phi([x,y,z],t)| + F_{AS}([x,y,z]) \cdot \nabla \Phi([x,y,z],t) = 0 \qquad (30)$$

The speed function is now defined based on voxels' intensities, $V(x, y, z)$, to reduce speed at locations with higher intensities. Therefore,

$$F_{AV}([x,y,z]) = \exp(-K_2 \|\nabla(G_\sigma * V(x,y,z))\|) \qquad (31)$$

where $G_\sigma$ is a Gaussian smoothing filter with standard deviation, $\sigma$, and $K_2$ is a constant that controls the rate of speed reduction. This definition provides a smooth reduction in the speed of Level-Set change, as the front approaches object boundaries. The term $F_{AS}(x, y, z)$ also is driven as follows,

$$F_{AS}([x, y, z]) = K_1 \nabla \left\| \nabla (G_\sigma * V(x, y, z)) \right\| \qquad (32)$$

Homogeneous regions have high values of $F_{AV}([x, y, z])$ leading to even growth of the Front while $F_{AS}(x, y, z)$ is small in these regions. When the Front approaches the boundary region, $F_{AV}([x, y, z])$ decreases while $F_{AS}(x, y, z)$ increases, thereby pushing the Front toward the correct boundary. Equation (32) can thus be used to update $\Phi([x, y, z],t)$ iteratively (step 655). As long as the exit condition is not satisfied (step 660), $\Phi([x, y, z],t)$ is continuously updated. This update procedure is iteratively repeated for a few iterations, $n_{iter}$. Once the iterative loop exits, the segmented 3D fluid region is displayed (step 670).

<u>Final Decision Making on Medical Condition</u>

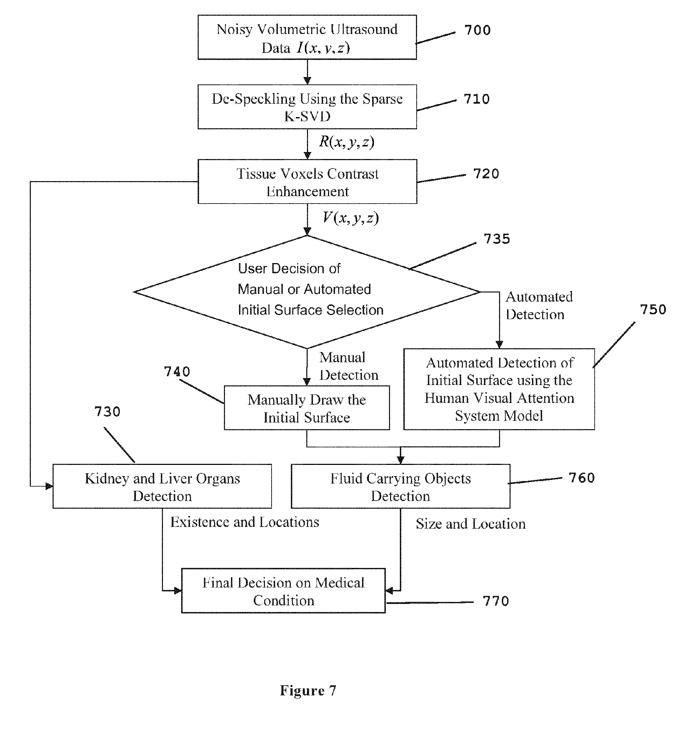

Figure 7 is a flowchart of the various steps in a process according to another aspect of the invention. The overall process of the invention is illustrated in Figure 7 with the final step being that of deciding whether the segmented fluid region is a fluid-carrying body organ or free blood fluid. If the detected fluid region is free blood fluid, the medical condition of internal bleeding is sent to the user so that immediate action may be taken. Inputs for this final decision step are the location of detected organs and the location and size of the segmented free fluid region. The anatomical knowledge, detected location of the kidney and liver, and the size and location of the segmented fluid region are applied to make the final decision.

For clarity, the process in Figure 7 starts with noisy volumetric ultrasound data (step 700). This data is then de-speckled or denoised using K-Singular Value Decomposition (step 710). The resulting data set then has its contrast enhanced (step 720). The enhanced contrast dataset is processed to find the image or a kidney, a liver, or other organs within the dataset (step 730). The same enhanced contrast dataset is also sent so that a user can determine whether to manually or automatically initial surface selection (step 735). If the user opts for manual entry, the user manually draws the initial surface (step 740). Alternatively, the initial surface can be automatically detected using the human visual attention system model (Step 750). Once the initial surface has been detected, whether manually or automatically, the size and location of the fluid carrying objects are determined (step 760). Once the size and location of the fluid carrying objects have been determined or once the kidney, liver, and other organs have been detected, step 770 is the final decision on the medical condition. Step 770 determines whether the area in the ultrasound image is a fluid filled cavity or whether the area is an internal organ.

As can be imagined, the methods and processes disclosed in this document may be used on any ultrasound imaging data derived from an ultrasound image of any mammal's body part. In one implementation, the various aspects of the invention may be used in both portable and non-portable medical equipment designed for use under less than ideal conditions such as remote locations, battlefield locations, mobile surgical hospitals, and disaster-stricken locations. In another aspect, the methods and processes described above may be implemented and used in non-portable equipment or equipment designed for a fixed installation. Such fixed equipment may be useful for detecting fluid accumulation in patients as well as for distinguishing internal organs from fluid accumulation in both trauma and non-trauma patients.

It should also be noted that while the above discussion mostly relates to 3D ultrasound imaging, the methods and processes discussed above may also be used on equipment which uses X-ray computed tomography (X-ray CT) or magnetic resonance imaging (MRI) technology.

<u>Implementation and Development of the Invented System</u>

The system can be implemented by computer programming software, such as MATLAB™, in conjunction with suitable computer hardware. All described steps and methods can be developed as individual modules and a designed GUI (graphical user interface) can combine these modules to form a unique toolbox to handle the detection procedure from the beginning to the final step. The GUI provides 3D display capability with the flexibility to move between x-plane, y-plane and z-plane and it also displays the 3D segmented objects.

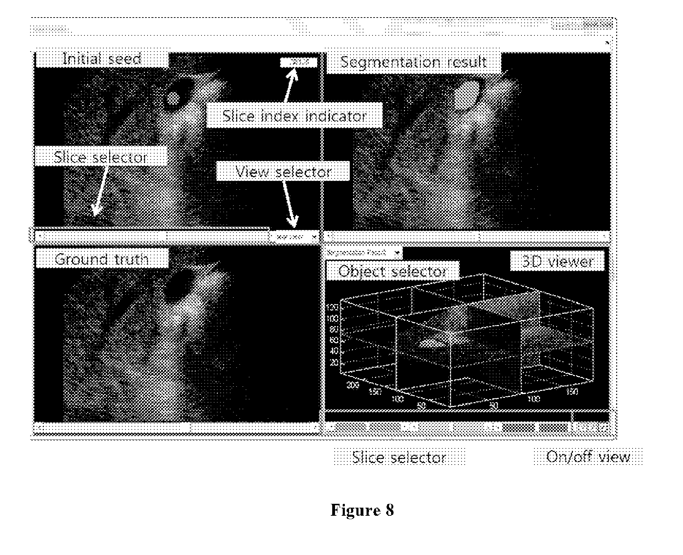

A view panel of a developed GUI according to another aspect of the invention is shown in Figure 8. Figure 8 also shows an automatically detected fluid region in the volumetric data.

For further reference and clarity regarding the varying aspects of the invention, the following references may be consulted:

B. J. C. and D. C., "Adaptive filtering for reduction of speckle in ultrasound pulse-echo images,," Ultrasonics, vol.

1, pp. 41-44, 1986.

G. A, G. J., M. S. and C. K. Deepika, "De-speckling of Medical Ultrasound Images using Wiener Filter and Wavelet Transform," International Journal of Electronics & Communication Technology, vol. 2, no. 3, pp. 21-24, 2011.

L. T., M. W. N. and A. P. L., "An adaptive weighted median filter for speckle suppression in medical ultrasonic images," IEEE Transaction on Circuits and Systems, vol. 36, pp. 129-135, 1989.

E. P. Simoncelli and A. E. H., "Noise removal via Bayesian wavelet coring," in Third International Conference on Image Processing, 1996.

P. Taya, S. T. Acton and J. A. Hossack, "A wavelet thresholding method to reduce ultrasound artifacts," Computerized Medical Imaging and Graphics, vol. 35, pp. 42-50, 2011.

Y. Yu and S. Acton, "Speckle reducing anisotropic diffusion," IEEE Transactions on Image Processing, , vol. 11, no. 11, pp. 1260-1270, 2002.

S. Kalaivani and R. Wahidabanu, "Condensed anisotropic diffusion for speckle reducton and enhancement in ultrasonography," EURASIP Journal on Image and Video Processing, vol. 2012, no. 12, pp. 1-17, 2012.

B. Burgeth, S. Didas, L. Florack and J. Weickert, "A generic approach to diffusion filtering of matrix-fields," Computing, vol. 81, no. 2, pp. 179-197, 2007.

P. C. Tay, C. D. Garson, S. T. Acton and J. A. Hossack, "Ultrasound Despeckling for Contrast Enhancement," IEEE Transactions on Image Processing, vol. 19, no. 7, pp. 1847-1860, 2010.

R. Rubinstein, M. Zibulevsky and M. Elad, "Double sparsity: learning sparse dictionaries for sparse signal approximation," IEEE Transactions on Signal Processing, vol. 58, no. 3, p. 1553, 2010.

A. Belaid, D. Boukerroui, Y. Maingourd and J. F. Lerallut, "Phase-Based Level Set Segmentation of Ultrasound Images," Information Technology in Biomedicine, IEEE Transactions on, vol. 5, no. 1, pp. 138-147, 2011.

E. Ukwatta, J. Awad, A. Ward, D. Buchanan, G. Parraga and A. Fenster, "Coupled level set approach to segment carotid arteries from 3D ultrasound images," in Biomedical Imaging: From Nano to Macro, 2011 IEEE International Symposium on, 2011.

M. Wahba, "An automated modified region growing technique for prostate segmentation in trans-rectal ultrasound images," ProQuest Dissertations and Theses, 2009.

R. J. Schneider, D. P. Perrin, N. V. Vasilyev, G. R. Marx, P. J. d. Nido and R. D. Howe, "Mitral Annulus Segmentation From 3D Ultrasound Using Graph Cuts," IEEE Transactions on Medical Imaging, vol. 29, no. 9, pp. 1676-1687, 2010.

R. Juang, E. McVeigh, B. Hoffmann, D. Yuh and P. Burlina, "Automatic segmentation of the left-ventricular cavity and atrium in 3D ultrasound using graph cuts and the radial symmetry transform," in IEEE International Symposium on Biomedical Imaging: From Nano to Macro, 2011, 2011.

X. Yang, W. He, J. Jin, X. Zhang, M. Yuchi and M. Ding, "A hybrid method to segment common carotid arteries from 3D ultrasound images," in IEEE-EMBS International Conference on Biomedical and Health Informatics (BHI), 2012, 2012.

R. Shekhar, R. M. Cothren, D. G. Vince, S. Chandra, J. D. Thomas and J. F. Cornhill, "Three-dimensional segmentation of luminal and adventitial borders in serial intravascular ultrasound images," Computerized Medical Imaging and Graphics, vol. 23, no. 6, pp. 299-309, 1999.

R. Whitaker, "A Level-Set Approach to 3D Reconstruction from Range Data," International Journal of Computer Vision, vol. 29, no. 3, pp. 203-231, 1998.

K. Mardia and T. J. Hainsworth, "A spatial thresholding method for image segmentation,," IEEE Transactions on Pattern Analysis and Machine Intelligence, vol. 10, no. 6, pp. 919-927, 1988.

L. Itti, C. Koch and E. Niebur, "A Model of Saliency-Based Visual Attention for Rapid Scene Analysis," IEEE Transactions on Pattern Analysis and Machine Intelligence, vol. 20, no. 11, pp. 1254-1259, 1998.

A. Yezzi and S. Soatto, "Deformotion: Deforming motion, shape average and the joint registration and approximation of structures in images," International Journal of Computer Vision, vol. 53, pp. 153-167, 2003.

M. R., J. A. Sethian and B. C. Vemuri, "Shape modeling with front propagation: a level set approach," IEEE Transactions on Pattern Analysis and Machine Intelligence, vol. 17, no. 2, pp. 158-175, 1995.

C. Revol and M. Jourlin, "A new minimum variance region growing algorithm for image segmentation," Pattern Recognition Letters, vol. 18, pp. 249-258, 1997.

P. Soille, Morphological Image Analysis: Principles and Applications, 1999, pp. 173-174.

A. K. Jain, Fundamental of Digital Image Processing., NJ: Prentice-Hall, 1989.

A. B. Watson, "Image compression using the Discrete Cosine Transform," Mathematica Jounral, vol. 4, no. 1, pp. 81-88, 1994.

M. Elad and M. Aheron, "Image Denoising Via Learned Dictionaries and Sparse representation," in Proceedings of the 2006 IEEE Computer Society Conference on Computer Vision and Pattern Recognition (CVPR '06), 2006.

Y. C. Pati, R. R. and K. P. S., "Orthogonal Matching Pursuit: recursive function approximation with applications to wavelet decomposition," Conference Record of The Twenty-Seventh Asilomar Conference on Signals, Systems and Computers,, vol. 1, pp. 40-44, 1993.

D. E. Lloyd, Automatic target classification using moment variants of iamge shapes, Franborough, UK, Rep. RAE IDN AW126, 1985.

B. Mory, O. Somphone, R. Prevost and R. Ardon, Template Deformation with User Constraints for Live 3D Interactive Surface Extraction, Toronto, Ontario, 2011.

D. Terzopoulos, A. Witkin and M. Kass, "Constrains on deformable models: recovering 3d shape and nonrigid motion," Artificial Intelligence, vol. 36, no. 1, pp. 91-123, 1988.

J. Ahlberg, "Active contours in three dimensions," Master's thesis, Linkping University, Computer Vision, The Institute of Technology , 1996.

M. Aheron, M. Elad and A. Bruckstein, "K-SVD: An Algorithm for Designing Overcomplete Dictionaries for Sparse Representation,," IEEE Transactions on Signal Processing, vol. 54, no. 12, pp. 4311-4322, 2006.

C. Koch and S. Ulman, "Shifts in selective visual attention: towards the underlying neural circuitry," Human Neurobiology, vol. 4, pp. 219-227, 1985.

L. Itti, C. Koch and E. Niebur, "A Model of Saliency-Based Visual Attention for Rapid Scene Analysis," IEEE Transactions on Pattern Analysis and Machine Intelligence, vol. 20, no. 11, pp. 1254-1259, 1998.

The method steps of the invention may be embodied in sets of executable machine code stored in a variety of formats such as object code or source code. Such code is described generically herein as programming code, or a computer program for simplification. Clearly, the executable machine code may be integrated with the code of other programs, implemented as subroutines, by external program calls or by other techniques as known in the art.

The embodiments of the invention may be executed by a computer processor or similar device programmed in the manner of method steps, or may be executed by an electronic system which is provided with means for executing these steps. Similarly, an electronic memory means such computer diskettes, CD-ROMs, Random Access Memory (RAM), Read Only Memory (ROM) or similar computer software storage media known in the art, may be programmed to execute such method steps. As well, electronic signals representing these method steps may also be transmitted via a communication network.

Embodiments of the invention may be implemented in any conventional computer programming language. For example, preferred embodiments may be implemented in a procedural programming language (e.g."C") or an object oriented language (e.g."C++"). Alternative embodiments of the invention may be implemented as pre-programmed hardware elements, other related components, or as a combination of hardware and software components. Embodiments can be implemented as a computer program product for use with a computer system. Such implementations may include a series of computer instructions fixed either on a tangible medium, such as a computer readable medium (e.g., a diskette, CD-ROM, ROM, or fixed disk) or transmittable to a computer system, via a modem or other interface device, such as a communications adapter connected to a network over a medium. The medium may be either a tangible medium (e.g., optical or electrical communications lines) or a medium implemented with wireless techniques (e.g., microwave, infrared or other transmission techniques). The series of computer instructions embodies all or part of the functionality previously described herein. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (e.g., shrink wrapped software), preloaded with a computer system (e.g., on system ROM or fixed disk), or distributed from a server over the network (e.g., the Internet or World Wide Web). Of course, some embodiments of the invention may be implemented as a combination of both software (e.g., a computer program product) and hardware. Still other embodiments of the invention may be implemented as entirely hardware, or entirely software (e.g., a computer program product).

A person understanding this invention may now conceive of alternative structures and embodiments or variations of the above all of which are intended to fall within the scope of the invention as defined in the claims that follow.

## Claims

1. A method for detecting an area of fluid in volumetric data derived from an image of a mammal's body part, the method comprising:

   a) receiving volumetric data;
   b) denoising said volumetric data to result in denoised data;
   c) enhancing a contrast of tissue voxels in said denoised data;
   d) determining an initial surface of a volume presented as a dark area in said denoised data;
   e) determining if an internal organ is detected in said volume in said denoised data; and
   f) isolating said volume in said denoised data to determine a size and location of said volume.

2. A method according to claim 1 wherein step b) is performed using K-Singular Value Decomposition.

3. A method according to claim 1 wherein step c) is performed by labelling voxels as tissue or non-tissue using thresholding.

4. A method according to claim 1 wherein step d) comprises generating a plurality of feature maps, deriving conspicuity maps from said feature maps, deriving a saliency map from said conspicuity maps, using a location in said saliency map as a center point for said initial surface.

5. A method according to claim 1 wherein step e) comprises determining at least one predetermined shape for said internal organ, deforming said at least one shape to determine if said at least one predetermined shape has a fit in said volume in said denoised data.

6. A method according to claim 1 wherein step f) comprises initiating a shape for said volume as a sphere and iteratively evolving said sphere to determine a boundary of said volume, said boundary being a surface of said volume.

7. A method according to claim 6 further comprising converting said surface into a binary volume and interpolating points on said surface to create a closed surface for said volume.

8. A method according to claim 1 further comprising a step of:

g) determining internal bleeding is occurring in the event said dark area is determined to not be an internal organ.

9. A method according to claim 1 wherein step c) comprises adjusting an intensity of tissue voxels relative to an intensity of non-tissue voxels.

10. Computer readable media having encoded thereon computer readable and computer executable instructions which, when executed, implements a method for detecting an area of fluid in volumetric data derived from an image of a mammal's body part, the method comprising:

    a) receiving volumetric data;
    b) denoising said volumetric data to result in denoised data;
    c) enhancing a contrast of tissue voxels in said denoised data;
    d) determining an initial surface of a volume presented as a dark area in said denoised data;
    e) determining if an internal organ is detected in said volume in said denoised data; and
    f) isolating said volume in said denoised data to determine a size and location of said volume.

11. Computer readable media according to claim 10 wherein step b) is performed using K-Singular Value Decomposition.

12. Computer readable media according to claim 10 wherein step c) comprises labelling voxels as tissue or non-tissue using thresholding and adjusting an intensity of tissue voxels relative to an intensity of non-tissue voxels.

13. Computer readable media according to claim 10 wherein step d) comprises generating a plurality of feature maps, deriving conspicuity maps from said feature maps, deriving a saliency map from said conspicuity maps, using a location in said saliency map as a center point for said initial surface.

14. Computer readable media according to claim 10 wherein step e) comprises determining at least one predetermined shape for said internal organ, deforming said at least one shape to determine if said at least one predetermined shape has a fit in said volume in said denoised data.

15. Computer readable media according to claim 10 wherein step f) comprises initiating a shape for said volume as a sphere and iteratively evolving said sphere to determine a boundary of said volume, said boundary being a surface of said volume.

**Figure 1**

De-Speckled Volumetric
Ultrasound Data $\hat{R}(x, y, z)$

GGMM-EM — 210

$$\overline{\theta} = \{\mu_{NT}, \sigma_{NT}, p_{NT}, \mu_T, \sigma_T, p_T\}$$

Create the matrix $P$
with equation (13) — 220

Calculate $t$ with
equation (14) — 230

Label voxels by
thresholding — 240

Update parameters
using ML method — 250

Converge — 255

No

Yes

Return Enhanced
Volume $V(x, y, z)$ — 260

**Figure 2**

Figure 3

Figure 4

| $n_s$ / $n_r$ | i-2 | i-1 | I | i+1 | i+2 |
|---|---|---|---|---|---|
| j-2 | 0 | 0 | $\beta$ | 0 | 0 |
| j-2 | 0 | $\beta$ | $-\alpha - 6\beta$ | $\beta$ | 0 |
| j | $\beta$ | $-\alpha - 6\beta$ | $4\alpha + 16\beta$ | $-\alpha - 6\beta$ | $\beta$ |
| j+1 | 0 | $\beta$ | $-\alpha - 6\beta$ | $\beta$ | 0 |
| j+2 | 0 | 0 | $\beta$ | 0 | 0 |

**Figure 5**

y

Create Initial Surface $S(r,s)$ — 610

Create Matrix $A$ — 615

$V(x,y,z)$

Calculate Balloon Force $f_{balloon}(x,y,z)$ for all voxels — 620

Find Balloon Force for Surface Sample $f_{balloon}(X(l,m),Y(l,m),Z(l,m))$ by Linear Interpolation — 623

— 630

Update Surface Samples $X(l,m),Y(l,m),Z(l,m)$ using equation (28)

No

Converge — 635

625

Yes

Convert Surface $S(s,r)$ into Binarized Volume $B(x,v,z)$ — 640

Create initial level-set function $\Phi([x,v,z],0)$ — 645

Calculate $F_{AV}([x,y,z])$ and $F_{AS}(x,y,z)$ for all voxels — 650

Update $\Phi([x,y,z],t)$ using equation (32)

655

Yes

$iter < n$ — 660

No

Display the segmented 3D Fluid Region — 670

Figure 6

Figure 7

Figure 8

Europäisches
Patentamt
European
Patent Office
Office européen
des brevets

# EUROPEAN SEARCH REPORT

Application Number

EP 14 15 1886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/024315 A1 (CHALANA VIKRAM [US] ET AL) 5 February 2004 (2004-02-05)<br>* abstract *<br>* figures 1-9 *<br>* paragraph [0011] - paragraph [0016] *<br>* paragraph [0028] - paragraph [0073] *<br>----- | 1-6,8-15 | INV.<br>A61B8/08<br>G06T5/00<br>G06T7/00<br>A61B8/00 |
| X | MARSOUSI M ET AL: "A multi-steps segmentation approach for 3D ultrasound images using the combination of 3D-Snake and Level-Set",<br>2013 18TH INTERNATIONAL CONFERENCE ON DIGITAL SIGNAL PROCESSING (DSP), IEEE,<br>2 July 2013 (2013-07-02), pages 1-4,<br>XP032498435,<br>ISSN: 1546-1874, DOI:<br>10.1109/ICDSP.2013.6622693<br>[retrieved on 2013-10-06]<br>* abstract *<br>* figure 2 *<br>* Sections I. and II. *<br>----- | 1,5-7,<br>10,14,15 | |
| A | US 2008/267468 A1 (GEIGER PAUL [CA] ET AL) 30 October 2008 (2008-10-30)<br>* abstract *<br>* paragraph [0049] - paragraph [0053] *<br>----- | 2,11 | TECHNICAL FIELDS SEARCHED (IPC)<br><br>A61B<br>G06T |
| A | US 2008/139938 A1 (YANG FUXING [US] ET AL) 12 June 2008 (2008-06-12)<br>* abstract *<br>* paragraph [0089] *<br>----- | 3,12 | |

The present search report has been drawn up for all claims

| Place of search | Date of completion of the search | Examiner |
|---|---|---|
| Munich | 3 July 2014 | Moehrs, Sascha |

EPO FORM 1503 03.82 (P04C01)

**ANNEX TO THE EUROPEAN SEARCH REPORT
ON EUROPEAN PATENT APPLICATION NO.**                    EP 14 15 1886

This annex lists the patent family members relating to the patent documents cited in  the above-mentioned European search report.
The members are as contained in the European Patent Office EDP file on
The European Patent Office is in no way liable for these particulars which are merely given for the purpose of information.

03-07-2014

| Patent document cited in search report | | Publication date | Patent family member(s) | | Publication date |
|---|---|---|---|---|---|
| US 2004024315 | A1 | 05-02-2004 | AT | 512628 T | 15-07-2011 |
| | | | AU | 2003261357 A1 | 23-02-2004 |
| | | | CA | 2534287 A1 | 12-02-2004 |
| | | | EP | 1538986 A2 | 15-06-2005 |
| | | | JP | 2005534462 A | 17-11-2005 |
| | | | JP | 2010207596 A | 24-09-2010 |
| | | | US | 2004024315 A1 | 05-02-2004 |
| | | | WO | 2004012584 A2 | 12-02-2004 |
| US 2008267468 | A1 | 30-10-2008 | CA | 2666313 A1 | 08-05-2008 |
| | | | EP | 2080169 A1 | 22-07-2009 |
| | | | JP | 5362573 B2 | 11-12-2013 |
| | | | JP | 2010505558 A | 25-02-2010 |
| | | | US | 2008267468 A1 | 30-10-2008 |
| | | | WO | 2008052312 A1 | 08-05-2008 |
| US 2008139938 | A1 | 12-06-2008 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7920731 B
- US 7803116 B
- US 8520947 B
- US 6561980 B
- US 6385332 B
- US 6719696 B
- US 6482160 B
- US 743490 A

**Non-patent literature cited in the description**

- **B. J. C. ; D. C.** Adaptive filtering for reduction of speckle in ultrasound pulse-echo images. *Ultrasonics,* 1986, vol. 1, 41-44
- **G. A, G. J. ; M. S. ; C. K. DEEPIKA.** De-speckling of Medical Ultrasound Images using Wiener Filter and Wavelet Transform. *International Journal of Electronics & Communication Technology,* 2011, vol. 2 (3), 21-24
- **L. T., M. W. N. ; A. P. L.** An adaptive weighted median filter for speckle suppression in medical ultrasonic images. *IEEE Transaction on Circuits and Systems,* 1989, vol. 36, 129-135
- **E. P. SIMONCELLI ; A. E. H.** Noise removal via Bayesian wavelet coring. *Third International Conference on Image Processing,* 1996
- **P. TAYA ; S. T. ACTON ; J. A. HOSSACK.** A wavelet thresholding method to reduce ultrasound artifacts. *Computerized Medical Imaging and Graphics,* 2011, vol. 35, 42-50
- **Y. YU ; S. ACTON.** Speckle reducing anisotropic diffusion. *IEEE Transactions on Image Processing,* 2002, vol. 11 (11), 1260-1270
- **S. KALAIVANI ; R. WAHIDABANU.** Condensed anisotropic diffusion for speckle reducton and enhancement in ultrasonography. *EURASIP Journal on Image and Video Processing,* 2012, vol. 2012 (12), 1-17
- **B. BURGETH ; S. DIDAS ; L. FLORACK ; J. WEICKERT.** A generic approach to diffusion filtering of matrix-fields. *Computing,* 2007, vol. 81 (2), 179-197
- **P. C. TAY ; C. D. GARSON ; S. T. ACTON ; J. A. HOSSACK.** Ultrasound Despeckling for Contrast Enhancement. *IEEE Transactions on Image Processing,* 2010, vol. 19 (7), 1847-1860
- **R. RUBINSTEIN ; M. ZIBULEVSKY ; M. ELAD.** Double sparsity: learning sparse dictionaries for sparse signal approximation. *IEEE Transactions on Signal Processing,* 2010, vol. 58 (3), 1553
- **A. BELAID ; D. BOUKERROUI ; Y. MAINGOURD ; J. F. LERALLUT.** Phase-Based Level Set Segmentation of Ultrasound Images. *Information Technology in Biomedicine, IEEE Transactions,* 2011, vol. 5 (1), 138-147
- **E. UKWATTA ; J. AWAD ; A. WARD ; D. BUCHANAN ; G. PARRAGA ; A. FENSTER.** Coupled level set approach to segment carotid arteries from 3D ultrasound images. *Biomedical Imaging: From Nano to Macro, 2011 IEEE International Symposium,* 2011
- **M. WAHBA.** An automated modified region growing technique for prostate segmentation in trans-rectal ultrasound images. *ProQuest Dissertations and Theses,* 2009
- **R. J. SCHNEIDER ; D. P. PERRIN ; N. V. VASILYEV ; G. R. MARX ; P. J. D. NIDO ; R. D. HOWE.** Mitral Annulus Segmentation From 3D Ultrasound Using Graph Cuts. *IEEE Transactions on Medical Imaging,* 2010, vol. 29 (9), 1676-1687
- **R. JUANG ; E. MCVEIGH ; B. HOFFMANN ; D. YUH ; P. BURLINA.** Automatic segmentation of the left-ventricular cavity and atrium in 3D ultrasound using graph cuts and the radial symmetry transform. *IEEE International Symposium on Biomedical Imaging: From Nano to Macro, 2011,* 2011
- **X. YANG ; W. HE ; J. JIN ; X. ZHANG ; M. YUCHI ; M. DING.** A hybrid method to segment common carotid arteries from 3D ultrasound images. *IEEE-EMBS International Conference on Biomedical and Health Informatics (BHI), 2012,* 2012
- **R. SHEKHAR ; R. M. COTHREN ; D. G. VINCE ; S. CHANDRA ; J. D. THOMAS ; J. F. CORNHILL.** Three-dimensional segmentation of luminal and adventitial borders in serial intravascular ultrasound images. *Computerized Medical Imaging and Graphics,* 1999, vol. 23 (6), 299-309
- **R. WHITAKER.** A Level-Set Approach to 3D Reconstruction from Range Data. *International Journal of Computer Vision,* 1998, vol. 29 (3), 203-231

- **K. MARDIA ; T. J. HAINSWORTH.** A spatial thresholding method for image segmentation. *IEEE Transactions on Pattern Analysis and Machine Intelligence,* 1988, vol. 10 (6), 919-927
- **L. ITTI ; C. KOCH ; E. NIEBUR.** A Model of Saliency-Based Visual Attention for Rapid Scene Analysis. *IEEE Transactions on Pattern Analysis and Machine Intelligence,* 1998, vol. 20 (11), 1254-1259
- **A. YEZZI ; S. SOATTO.** Deformotion: Deforming motion, shape average and the joint registration and approximation of structures in images. *International Journal of Computer Vision,* 2003, vol. 53, 153-167
- **M. R. ; J. A. SETHIAN ; B. C. VEMURI.** Shape modeling with front propagation: a level set approach. *IEEE Transactions on Pattern Analysis and Machine Intelligence,* 1995, vol. 17 (2), 158-175
- **C. REVOL ; M. JOURLIN.** A new minimum variance region growing algorithm for image segmentation. *Pattern Recognition Letters,* 1997, vol. 18, 249-258
- **P. SOILLE.** *Morphological Image Analysis: Principles and Applications,* 1999, 173-174
- **A. K. JAIN.** Fundamental of Digital Image Processing. Prentice-Hall, 1989
- **A. B. WATSON.** Image compression using the Discrete Cosine Transform. *Mathematica Jounral,* 1994, vol. 4 (1), 81-88
- **M. ELAD ; M. AHERON.** Image Denoising Via Learned Dictionaries and Sparse representation. *Proceedings of the 2006 IEEE Computer Society Conference on Computer Vision and Pattern Recognition (CVPR '06),* 2006
- **Y. C. PATI, R. R. ; K. P. S.** Orthogonal Matching Pursuit: recursive function approximation with applications to wavelet decomposition. *Conference Record of The Twenty-Seventh Asilomar Conference on Signals, Systems and Computers,* 1993, vol. 1, 40-44
- **D. E. LLOYD.** *Automatic target classification using moment variants of iamge shapes,* 1985
- **B. MORY ; O. SOMPHONE ; R. PREVOST ; R. AR-DON.** *Template Deformation with User Constraints for Live 3D Interactive Surface Extraction,* 2011
- **D. TERZOPOULOS ; A. WITKIN ; M. KASS.** Constrains on deformable models: recovering 3d shape and nonrigid motion. *Artificial Intelligence,* 1988, vol. 36 (1), 91-123
- Active contours in three dimensions. **J. AHLBERG.** Master's thesis. The Institute of Technology, 1996
- **M. AHERON ; M. ELAD ; A. BRUCKSTEIN.** K-SVD: An Algorithm for Designing Overcomplete Dictionaries for Sparse Representation. *IEEE Transactions on Signal Processing,* 2006, vol. 54 (12), 4311-4322
- **C. KOCH ; S. ULMAN.** Shifts in selective visual attention: towards the underlying neural circuitry. *Human Neurobiology,* 1985, vol. 4, 219-227